# EUROPÄISCHE PATENTSCHRIFT

(11) **EP 3 600 470 B1**
(45) Veröffentlichungstag und Bekanntmachung des Hinweises auf die Patenterteilung: **27.08.2025**
(21) Anmeldenummer: 18711339.4
(22) Anmeldetag: 16.03.2018
(51) Int. Cl.: A61M 1/00, A61M 3/02, F04B 43/12, F04B 23/04

(54) **VORRICHTUNG MIT EINER KOPPELBAREN PERISTALTIKPUMPENEINHEIT**
DEVICE WITH A DOCKABLE PERISTALTIC PUMP UNIT
DISPOSITIF COMPRENANT UNE UNITÉ DE POMPE PÉRISTALTIQUE POUVANT ÊTRE COUPLÉE

(30) Priorität: 23.03.2017 EP 17162577
(43) Veröffentlichungstag der Anmeldung: 05.02.2020
(73) Patentinhaber: Medela Holding AG, 6340 Baar (CH)
(72) Erfinder: BANNWART, Lukas, 6343 Rotkreuz (CH)
(74) Vertreter: Grünecker Patent- und Rechtsanwälte PartG mbB
(86) Internationale Anmeldenummer: PCT/EP2018/056705
(87) Internationale Veröffentlichungsnummer: WO 2018/172217

(56) Entgegenhaltungen:
- EP-B1- 1 743 100
- US-A- 3 421 447
- US-A- 5 044 902
- US-A1- 2009 074 597
- US-B1- 6 280 440

## Beschreibung

### TECHNISCHES GEBIET

Die vorliegende Erfindung betrifft eine Vorrichtung zum Zuführen und/oder Absaugen einer fluiden Substanz zu oder von einem menschlichen oder tierischen Körper sowie ein Anschlussteil einer derartigen Vorrichtung. Derartige Vorrichtungen werden insbesondere im medizinischen Bereich verwendet, beispielsweise in der mit Instillation oder Irrigation kombinierten Unterdruck-Wundtherapie, in der Augenchirurgie oder bei der Fettabsaugung.

### STAND DER TECHNIK

Im medizinischen Bereich gibt es vielfältige Anwendungen, bei denen einerseits Körperfluide oder Sekrete aus Körperkavitäten oder Wunden mittels einer Pumpe abgesaugt und andererseits eine Substanz dem Körper zugeführt werden. Mögliche Anwendungsgebiete betreffen insbesondere die mit Instillation kombinierte Unterdruck-Wundtherapie, die Augenchirurgie und die Liposuktion (Fettabsaugung). Je nach Anwendung erfolgen die Absaugung und die Zuführung dabei gleichzeitig, nacheinander und/oder abwechslungsweise intermittierend. Selbstverständlich gibt es auch viele medizinische Anwendungen, bei denen nur eine Absaugung von Fluiden aus dem Körper stattfindet, ohne dass eine Substanz zugeführt wird. Genauso gibt es zudem Anwendungen, bei denen nur eine Substanz dem Körper zugeführt wird, ohne dass notwendigerweise auch eine Absaugung stattfinden muss.

Falls eine fluide Substanz abgesaugt wird, kann es sich zum Beispiel um Sekrete handeln, die in einem Wundbereich vom Körper abgesondert werden, oder um Instillations- oder Spülflüssigkeit oder um beides davon.

Bei der zuzuführenden Substanz kann es sich beispielsweise um eine physiologische oder nicht physiologische Kochsalzlösung, ein Arzneimittel oder um ein Gemisch davon handeln. Die Substanz kann zum Beispiel zur Förderung der Wundheilung, zur Verhinderung von Infektionen oder zur lokalen Anästhesie dienen. Das Zuführen der Substanz kann somit zur Spülung oder therapeutischen, diagnostischen und/oder präventiven Zwecken dienen.

Oft wird zum Zuführen der Substanz ähnlich wie bei der herkömmlichen Infusion ein mit der zuzuführenden Substanz gefüllter Flüssigkeitsbeutel oder eine Flasche erhöht über der zu behandelnden Körperstelle angeordnet, so dass die Substanz aufgrund des hydrostatischen Druckes durch eine Zuführleitung der zu behandelnden Stelle zugeführt wird. Separat dazu werden oft Körperfluide von einer Vakuumpumpe via eine entsprechende Leitung abgesaugt.

Um eine bessere Einstellung und Regelung beim Zuführen der Substanz zu ermöglichen, und/oder um unabhängig von der Anordnung und insbesondere der Höhenlage des mit der Substanz gefüllten Flüssigkeitsbehälters zu sein, sind auch Systeme hinlänglich bekannt, bei denen die Zuführung der Substanz zum Körper mittels einer Pumpe, insbesondere einer sog. Peristaltik- oder Schlauchpumpe erfolgt. Auch die Absaugung von fluiden Substanzen vom Körper kann mittels einer Peristaltikpumpe bewerkstelligt werden.

Gattungsgemässe Peristaltik- bzw. Schlauchpumpen sind beispielsweise in den Dokumenten EP 0 130 374 A2, DE 42 14 916 A1, DE 10 2005 055 013 B3, WO 94/03728, US 4,181,476, WO 91/14100 und US 5,927,956 offenbart.

Die DE 38 12 109 A1 offenbart eine Schlauchpumpe, welche jedoch zur Zuführung eines Reinigungsmittels bei Haushaltsgeräten ausgebildet ist.

In der US 2014/0163487 ist ein Gerät mit zwei Pumpen offenbart, wobei hier der Pumpenkopf einer Peristaltikpumpe, welche zum Zuführen einer Substanz zum Körper dient, auf der Aussenseite des Pumpaggregatgehäuses angeordnet ist. Ein Flüssigkeitsbehälter, welcher zur Aufnahme einer Instillationsflüssigkeit dient, ist an das Pumpaggregatgehäuse anschliessbar. Am Flüssigkeitsbehälter ist eine Schlauchführung ausgebildet, aufgrund welcher der Pumpenkopf, wenn der Behälter am Pumpaggregatgehäuse angeschlossen ist, eine entsprechende Pumpwirkung auf den aus dem Behälterinneren herausführenden Instillationsschlauch ausübt, um so die Instillationsflüssigkeit zum Körper hin zu pumpen.

Für Reinigungszwecke und insbesondere um die Lebensdauer des Schlauches während der Lagerung und beim Transport zu verlängern, kann es wünschenswert sein, die Belastung des Schlauches zu reduzieren, wenn die Schlauchpumpe nicht in Betrieb ist.

Die US 4,218,197 und die EP 0 388 269 B1 offenbaren deshalb jeweils eine in radialer Richtung variable Positionierung von Rollen am Pumpenkopf. Indem die Rollen radial nach innen verschoben werden, wird der auf den Schlauch ausgeübte Druck verringert.

Bei der in der EP 1 743 100 B1 offenbarten Vorrichtung wird bei der Ankopplung des Pumpenkopfes an eine Antriebseinheit ein Spreizteil eines Kopplungselements zwischen die in radialer Richtung bewegbar am Pumpenkopf angebrachten Rollenelemente geschoben. Die Rollenelemente werden dadurch in radialer Richtung nach aussen gegen den darum herum gelegten Schlauch gepresst. Die Übertragung des Drehmoments vom Kopplungselement auf den Pumpenkopf erfolgt via Mitnehmerstifte, welche in Ausnehmungen eingreifen, die entsprechend an einer die Rollenelemente verbindenden Halteplatte des Pumpenkopfes vorgesehen sind. Diese Lösung hat den Nachteil, dass bei der Übertragung des Drehmoments vom Kopplungselement auf den Pumpenkopf keine Über- bzw. Untersetzung der Drehzahl möglich ist.

In der CN 205681233 U und der CN 205117687 U sind Peristaltikpumpen offenbart, bei denen der Pumpenkopf durch drei mittels einer Verbindungsplatte miteinander verbundenen Rollenelemente gebildet wird. Die Rollenelemente sind zu einem gewissen Grad in radialer Richtung bewegbar und werden mittels eines Schaftes angetrieben, der zwischen die Rollenelemente geschoben wird und diese dadurch in radialer Richtung gegen den Schlauch presst. Die Übertragung des Drehmoments vom Schaft auf den Pumpenkopf erfolgt mittels Reibschluss. Um genau definierte Pumpeigenschaften zu erreichen, müssen die Rollenelemente aus hochwertigen Materialien und mit engen Herstelltoleranzen angefertigt werden.

Die DE 694 02 428 T2 und die US 6,280,440 B1 offenbaren jeweils eine Peristaltikpumpenkassette, bei welcher drei Rollen innerhalb einer von einem Schlauch gebildeten Schleife angeordnet sind. Bei Benutzung der Peristaltikpumpenkassette wird eine Antriebsachse zentral zwischen die Rollen vorgeschoben, wodurch diese radial nach aussen gegen den Schlauch gedrückt werden. Beim Antrieb erfolgt die Kraftübertragung jeweils von der Antriebsachse auf die Rollen mittels Reibung.

Die US 5,044,902 A schlägt eine Peristaltikpumpe vor, bei welcher frei drehbare, an einer rotierenden Scheibe angebrachte Rollen in das Innere von Abwälzrollen eingeschoben werden, um die Drehbewegung von der Scheibe auf Abwälzrollen zu übertragen. Gleichzeitig werden die Abwälzrollen von einer zentralen Rolle radial nach aussen gegen einen Schlauch gedrückt.

### DARSTELLUNG DER ERFINDUNG

Es ist eine Aufgabe der Erfindung, eine günstig herstellbare, aber doch effiziente Peristaltikpumpeneinheit anzugeben.

Zur Lösung dieser Aufgabe wird eine Vorrichtung vorgeschlagen, wie sie im Anspruch 1 angegeben ist. Ausserdem wird im Anspruch 14 ein Anschlussteil einer derartigen Vorrichtung angegeben. Vorteilhafte Ausgestaltungen der Erfindung sind in den abhängigen Ansprüchen angegeben.

Die vorliegende Erfindung stellt also eine Vorrichtung zum Zuführen und/oder Absaugen einer fluiden Substanz zu oder von einem menschlichen oder tierischen Körper zur Verfügung, aufweisend
eine Antriebseinheit mit einem Antrieb, wie insbesondere einem Motor;
ein an die Antriebseinheit anschliessbares Anschlussteil, welches eine Peristaltikpumpeneinheit mit einem Pumpenkopf, einer Schlauchführung sowie einem in der Schlauchführung eingelegten Schlauch aufweist, wobei der Pumpenkopf eines oder mehrere Rollenelemente aufweist, welche zum Abrollen auf dem Schlauch dienen, um dadurch die fluide Substanz durch den Schlauch hindurch zu befördern, insbesondere zum menschlichen oder tierischen Körper hin oder vom menschlichen oder tierischen Körper weg zu befördern; sowie
ein mit dem Pumpenkopf koppelbares Kopplungselement, welches bei bestimmungsgemässem Anschluss des Anschlussteils an die Antriebseinheit zur Übertragung einer Bewegung vom Antrieb auf den Pumpenkopf dient.

Der Pumpenkopf weist einen vom Kopplungselement entkoppelten Zustand auf, in welchem das oder die Rollenelemente keinen oder einen vergleichsweise geringen mechanischen Druck auf den Schlauch ausüben, und weist zudem einen mit dem Kopplungselement gekoppelten Zustand auf, in welchem das oder die Rollenelemente vom Kopplungselement mit einem vergleichsweise grossen mechanischen Druck gegen den Schlauch gepresst werden.

Das oder die Rollenelemente sind im entkoppelten Zustand innerhalb eines Bereiches frei und unabhängig voneinander bewegbar, der von dem in die Schlauchführung eingelegten Schlauch seitlich begrenzt ist. Die einzelnen Rollenelemente sind also nicht miteinander verbunden, sondern sind separat voneinander im erwähnten Bereich angeordnet. Bei den Rollenelementen handelt es sich somit um bzgl. ihrer Bewegungsfreiheit eigenständige Teile. Falls mehrere Rollenelemente vorhanden sind, können sich diese im entkoppelten Zustand in der Regel gegenseitig berühren. Durch das Weglassen einer gegenseitigen Verbindung der Rollenelemente verringert sich der Herstellungsaufwand und die Montage erheblich.

Das oder die Rollenelemente sind bevorzugt jeweils zylindrisch ausgebildet.

Das Kopplungselement weist einen ersten Verzahnungsbereich auf. Der Pumpenkopf weist ausserdem einen zweiten Verzahnungsbereich auf, welcher im gekoppelten Zustand derart in den ersten Verzahnungsbereich des Kopplungselements eingreift, dass eine Bewegung des Kopplungselements direkt auf den Pumpenkopf übertragen wird. Aufgrund des gegenseitigen Eingreifens des ersten und des zweiten Verzahnungsbereiches erfolgt die Übertragung des Drehmoments vom Kopplungselement auf den Pumpenkopf im Betrieb der Peristaltikpumpe auf eine besonders effiziente Weise und mit einem einstellbaren, klar definierten Über- bzw. Untersetzungsverhältnis. Das Drehmoment wird insbesondere direkt, das heisst nicht via ein Zwischenteil, vom Kopplungselement auf den Pumpenkopf und insbesondere auf das oder die unmittelbar auf den Schlauch einwirkenden Teile übertragen. Falls der Pumpenkopf durch mehrere Rollenelemente gebildet wird, ist eine gegenseitige Verbindung der Rollenelemente zur Einschränkung von deren Bewegungsspielraum bzw. zur Gewährleistung eines einheitlichen Bewegungsablaufs nicht zwingend notwendig. Selbst wenn sich die einzelnen Rollenelemente im Betrieb gegenseitig berühren, hat die dabei zwischen den Rollenelementen erzeugte Reibungskraft keinen Einfluss auf deren Bewegung, da der Kraftschluss vom Kopplungselement auf die einzelnen Rollenelemente wesentlich effizienter ist. Selbst bei sehr hohen Pumpleistungen und einem entsprechend im Schlauch hohen anliegenden Druck ist das Über- bzw. Untersetzungsverhältnis zwischen Kopplungselement und Pumpenkopf klar definiert.

In einer alternativen Ausführungsform kann die Übertragung der Bewegung vom Kopplungselement auf den Pumpenkopf und insbesondere auf das oder die Rollenelemente auch mittels reiner Reibungskraft erfolgen. Die entsprechenden Oberflächen des Kopplungselementes und des bzw. der Rollenräder, welche dann zum Beispiel zylindrisch ausgebildet sein können, sind dann vorteilhaft derart ausgebildet, dass sie einen hohen Reibungskoeffizient aufweisen.

Im gekoppelten Zustand presst das Kopplungselement also den Pumpenkopf bzw. Teile des Pumpenkopfes, wie insbesondere eines oder mehrere Rollenelemente, in radialer Richtung nach aussen gegen den Schlauch. Im Falle von mehreren Rollenelementen kann das Kopplungselement auch als Spreizelement bezeichnet werden. Im entkoppelten Zustand sind der Pumpenkopf bzw. dessen Teile im Vergleich zum gekoppelten Zustand aufgrund des Druckes des Schlauches radial nach innen verschoben. Das Schlauchmaterial ist deshalb kaum belastet, wodurch der Schlauch im Hinblick auf Transport und Lagerung eine grössere Lebensdauer hat, und/oder mit einer geringeren Wandstärke ausgebildet werden kann. Bei einer geringeren Wandstärke des üblicherweise aus Silikon hergestellten Schlauches verringert sich zudem die vom Antrieb im Betrieb zu leistende Arbeit.

Das oder die Rollenelemente können im entkoppelten Zustand jeweils ein gewisses seitliches Spiel haben.

Der Pumpenkopf und insbesondere dessen Rollenelemente werden bevorzugt im gekoppelten Zustand derart vom Kopplungselement gegen den Schlauch gepresst, dass sie sich im Vergleich zum entkoppelten Zustand für das menschliche Auge gut sichtbar und insbesondere elastisch verformen. Dadurch ergibt sich eine gewisse Federwirkung, wodurch eine klar definierte Anpresskraft auf den Schlauch selbst bei verhältnismässig grossen herstellungsbedingten Toleranzen erreicht wird.

Um beim Anschliessen des Anschlussteils an die Antriebseinheit das Ankoppeln des Kopplungselements am Pumpenkopf sowie eine kontinuierliche Erhöhung des Anpressdrucks auf den Schlauch zur erreichen, weist das Kopplungselement vorteilhaft einen konisch ausgebildeten Abschnitt, insbesondere Endabschnitt, auf.

Vorteilhaft weist das Kopplungselement zudem einen zylindrischen Abschnitt auf, welcher im gekoppelten Zustand zum Pressen des oder der Rollenelemente gegen den Schlauch dient. Das Anpressen erfolgt dann vorteilhaft nicht über allfällig vorhandene Verzahnungsbereiche.

Das Kopplungselement kann ein Teil der Antriebseinheit darstellen. Es kann aber auch ein Teil des Anschlussteils sein.

Beim Anschlussteil kann es sich insbesondere um einen Fluidbehälter zum Sammeln oder Bereitstellen eines Fluids handeln. Dieser kann ein Wegwerfteil darstellen, welches nach einmaligem Gebrauch entsorgt wird.

Das oder die Rollenelemente werden bevorzugt jeweils durch ein Hohlrad gebildet. Ein Hohlrad ist ein im Wesentlichen kreisrundes Objekt mit einer radial nach aussen hin gerichteten Aussenfläche und einer radial nach innen gerichteten Innenfläche. Auf der Aussen- und/oder Innenfläche ist üblicherweise ein Zahnkranz ausgebildet.

Der zweite Verzahnungsbereich kann somit durch jeweils einen an dem einen oder an den mehreren Hohlrädern ausgebildeten äusseren Zahnkranz gebildet werden.

Beim zweiten Verzahnungsbereich kann es sich aber auch um einen am Hohlrad ausgebildeten inneren Zahnkranz handeln. Bei einer derartigen Ausführungsform weist das Hohlrad bevorzugt ein zentral angeordnetes Umlaufelement auf, welches im gekoppelten Zustand am Kopplungselement anliegt.

Bevorzugt ist der Pumpenkopf als Ganzes im Spritzgussverfahren aus einem Kunststoff, insbesondere aus Polypropylen (PP), hergestellt. Er kann aber auch aus anderen insbesondere organischen Werkstoffen hergestellt sein.

Die vorliegende Erfindung bezieht sich ausserdem auf ein Anschlussteil einer Vorrichtung zum Zuführen und/oder Absaugen einer fluiden Substanz zu oder von einem menschlichen oder tierischen Körper, insbesondere auf ein Anschlussteil einer Vorrichtung entsprechend den oben erwähnten Erläuterungen. Das Anschlussteil weist eine Peristaltikpumpeneinheit mit einem Pumpenkopf, einer Schlauchführung sowie einem in der Schlauchführung eingelegten Schlauch auf, wobei der Pumpenkopf eines oder mehrere Rollenelemente aufweist, welche zum Abrollen auf dem Schlauch dienen, um dadurch die fluide Substanz durch den Schlauch hindurch zu befördern, insbesondere zum menschlichen oder tierischen Körper hin oder vom menschlichen oder tierischen Körper weg zu befördern. Der Pumpenkopf ist mit einem Kopplungselement koppelbar, welches zur Übertragung einer Bewegung von einem Antrieb auf den Pumpenkopf dient. Das oder die Rollenelemente üben in einem vom Kopplungselement entkoppelten Zustand keinen oder einen vergleichsweise geringen mechanischen Druck auf den Schlauch aus, sind aber mit dem Kopplungselement derart koppelbar ist, dass sie in einem gekoppelten Zustand vom Kopplungselement mit einem vergleichsweise grossen mechanischen Druck gegen den Schlauch gepresst werden. Das oder die Rollenelemente sind im entkoppelten Zustand innerhalb eines Bereiches frei und unabhängig voneinander bewegbar, der von dem in die Schlauchführung eingelegten Schlauch seitlich begrenzt ist.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1a: eine perspektivische Ansicht einer schematisch gezeigten Vorrichtung gemäss einer erfindungsgemässen ersten Ausführungsform mit einem Anschlussteil in Form eines Fluidsammelbehälters sowie einem Pumpaggregatgehäuse, wobei die Vorderwand des Pumpaggregatgehäuses weggelassen ist;
- Fig. 1b: eine zweite perspektivische Ansicht des Fluidsammelbehälters der in der Fig. 1a gezeigten Vorrichtung aus einem anderen Betrachtungswinkel;
- Fig. 1c: der in der Fig. 1b gezeigte Fluidsammelbehälter in derselben Ansicht, aber mit abgenommener Abdeckung;
- Fig. 1d: eine perspektivische Detailansicht der Peristaltikpumpeneinheit des in der Fig. 1c gezeigten Fluidsammelbehälters inkl. des Kopplungselements des in der Fig. 1a gezeigten Pumpaggregatgehäuses in entkoppeltem Zustand;
- Fig. 1e: eine perspektivische Detailansicht der Peristaltikpumpeneinheit des in der Fig. 1b gezeigten Fluidsammelbehälters inkl. des Kopplungselements des in der Fig. 1a gezeigten Pumpaggregatgehäuses in entkoppeltem Zustand;
- Fig. 1f: eine perspektivische Detailansicht der Peristaltikpumpeneinheit des in der Fig. 1c gezeigten Fluidsammelbehälters inkl. des Kopplungselements des in der Fig. 1a gezeigten Pumpaggregatgehäuses in gekoppeltem Zustand;
- Fig. 1g: eine perspektivische Detailansicht der Peristaltikpumpeneinheit des in der Fig. 1b gezeigten Fluidsammelbehälters inkl. des Kopplungselements des in der Fig. 1a gezeigten Pumpaggregatgehäuses in gekoppeltem Zustand;
- Fig. 1h: eine Draufsicht auf die Peristaltikpumpeneinheit des in der Fig. 1c gezeigten Fluidsammelbehälters inkl. des Kopplungselements des in der Fig. 1a gezeigten Pumpaggregatgehäuses in gekoppeltem Zustand;
- Fig. 2a: eine perspektivische Ansicht eines Anschlussteils in Form einer Peristaltikkassette einer Vorrichtung gemäss einer erfindungsgemässen zweiten Ausführungsform, in entkoppeltem Zustand;
- Fig. 2b: die in der Fig. 2a gezeigte Peristaltikkassette mit Pumpenkopf in entkoppeltem Zustand und mit abgenommener Abdeckung;
- Fig. 2c: die in der Fig. 2a gezeigte Peristaltikkassette mit abgenommener Abdeckung und ohne Pumpenkopf, Instillationsschlauch und Koppelungselement;
- Fig. 2d: die in der Fig. 2a gezeigte Peristaltikkassette in gekoppeltem Zustand;
- Fig. 2e: die in der Fig. 2a gezeigte Peristaltikkassette mit Pumpenkopf in gekoppeltem Zustand und mit abgenommener Abdeckung;
- Fig. 2f: eine perspektivische Ansicht der Abdeckung, des Kopplungselements sowie eines Teils des Pumpenkopfes der in der Fig. 2a gezeigte Peristaltikkassette in gekoppeltem Zustand;
- Fig. 3a: eine perspektivische Ansicht eines Anschlussteils sowie eines Kopplungselements einer Vorrichtung gemäss einer erfindungsgemässen dritten Ausführungsform, in entkoppeltem Zustand;
- Fig. 3b: das in der Fig. 3a gezeigte Anschlussteil in entkoppeltem Zustand und mit abgenommener Abdeckung;
- Fig. 3c: eine Draufsicht auf die Peristaltikpumpeneinheit des in der Fig. 3b gezeigten Anschlussteils in entkoppeltem Zustand und mit abgenommener Abdeckung;
- Fig. 3d: eine perspektivische Ansicht des in der Fig. 3a gezeigten Anschlussteils ohne Abdeckung, Pumpenkopf und Instillationsschlauch;
- Fig. 3e: eine perspektivische Ansicht des in der Fig. 3a gezeigten Anschlussteils sowie des in der Fig. 3a gezeigten Kopplungselements, in gekoppeltem Zustand;
- Fig. 3f: das in der Fig. 3e gezeigte Anschlussteil mit Koppelungselement in gekoppeltem Zustand und mit abgenommener Abdeckung;
- Fig. 3g: eine Draufsicht auf die Peristaltikpumpeneinheit des in der Fig. 3f gezeigten Anschlussteils mit Koppelungselement in gekoppeltem Zustand und mit abgenommener Abdeckung; sowie
- Fig. 3h: eine zentrale Querschnittsansicht entlang der Linie III-III durch das in der Fig. 3g gezeigte Anschlussteil mit Koppelungselement in gekoppeltem Zustand und mit abgenommener Abdeckung.

### BESCHREIBUNG BEVORZUGTER AUSFÜHRUNGSFORMEN

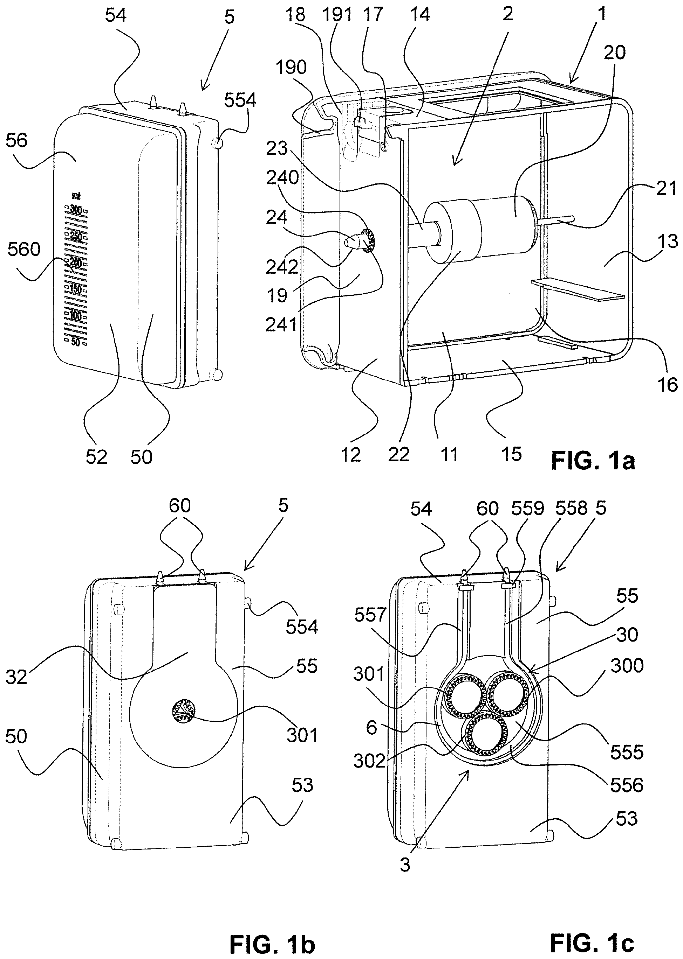

In den Figuren 1a bis 3h sind unterschiedliche Ausführungsformen von erfindungsgemässen Vorrichtungen bzw. von erfindungsgemässen Anschlussteilen gezeigt. Die in den Figuren 1a bis 3h gezeigten Vorrichtungen sind zur Instillations-/Irrigationsbehandlung von Wunden am menschlichen oder tierischen Körper geeignet, insbesondere in Kombination mit einer Unterdruckbehandlung. Dementsprechend beziehen sich die nachfolgenden Erläuterungen jeweils auf die Verwendung der Vorrichtungen Instillations-/Irrigationsbehandlung, gegebenenfalls in Kombination mit einer Unterdruckbehandlung. Es wäre grundsätzlich aber auch möglich, diese Vorrichtungen, bei entsprechend angepasster Auslegung, zum Beispiel für die Katheterspülung, die Augenchirurgie, die Fettabsaugung oder eine andere medizinische Anwendung zu verwenden.

Elemente mit einer identischen oder ähnlichen technischen Funktion und Wirkung sind in den Figuren 1a bis 3h bei den verschiedenen Ausführungsformen jeweils mit denselben Bezugszeichen versehen.

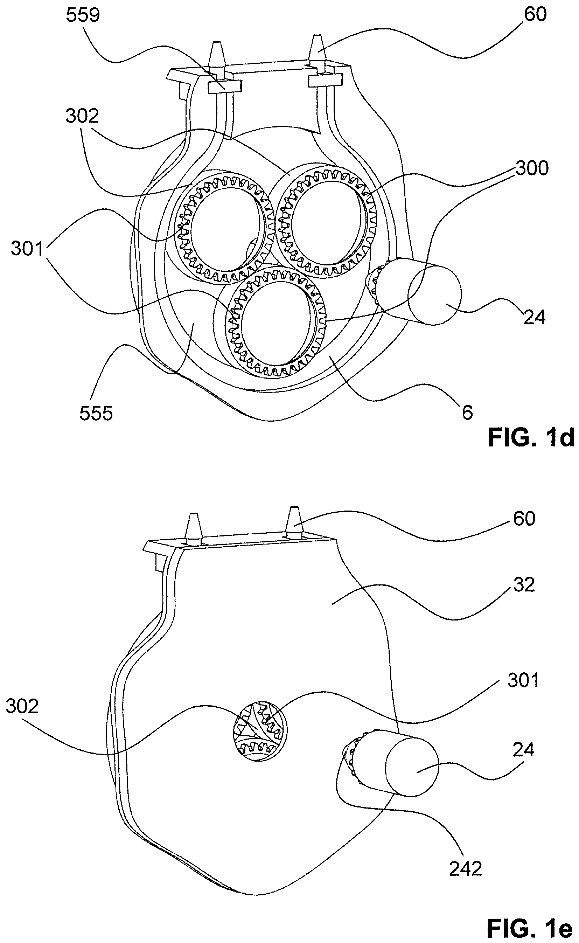

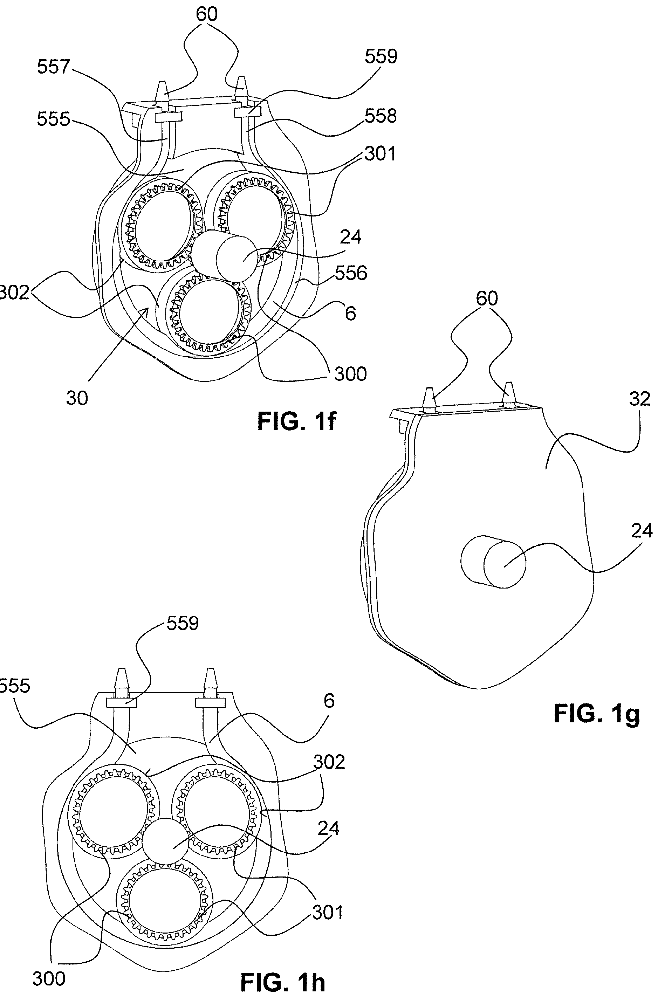

Die Vorrichtung der ersten erfindungsgemässen Ausführungsform, welche in den Figuren 1a bis 1h gezeigt ist, weist ein Pumpaggregatgehäuse 1 mit einem daran anschliessbaren Anschlussteil in Form eines Fluidsammelbehälters 5 (Figur 1) auf.

Während das Pumpaggregatgehäuse 1 inkl. seinem Innenleben üblicherweise für eine mehrmalige Verwendung und somit eine möglichst lange Lebensdauer ausgelegt ist, stellt der Fluidsammelbehälter in vielen Anwendungen insbesondere aus hygienischen Gründen einen Wegwerfartikel dar, der nach einmaliger, oft nur einige Tage oder Stunden dauernder Anwendung entsorgt wird.

Das Pumpaggregatgehäuse 1 hat eine insgesamt im Wesentlichen quaderförmige Gestalt mit einer in der Figur 1a nicht gezeigten Vorderwand, einer Rückwand 11, einer ersten Seitenwand 12 und einer zweiten Seitenwand 13 sowie einer oberen Wand 14 und einer unteren Wand 15. Die Vorderwand und die Rückwand 11 weisen je eine Wandkante auf, welche der dazwischen angeordneten ersten Seitenwand 12 vorstehen. Der Fluidsammelbehälter 5 ist zwischen diesen Wandkanten gehalten und dadurch einfach, aber trotzdem sicher und geschützt am Pumpaggregatgehäuse 1 befestigbar.

Zum Einhängen und Halten des Fluidsammelbehälters 5 am Pumpaggregatgehäuse 1 sind am Pumpaggregatgehäuse 1 Aufnahmehaken 190 vorgesehen, in welche entsprechend ausgebildete und angeordnete Zapfen 554 des Fluidsammelbehälters 5 eingreifen können. Das Pumpaggregatgehäuse 1 kann eine an einem federbelasteten Element angebrachte Rückhaltenase 191 aufweisen, welche zum Einschnappen in eine am Fluidsammelbehälter 5 entsprechend ausgebildete, aber in den Figuren nicht gezeigte Rastkerbe ausgebildet ist, um den Fluidsammelbehälter 5 am Pumpaggregatgehäuse 1 zu sichern. Um die Rastverbindung zwischen der Rückhaltenase 191 und der Rastkerbe zu lösen, kann das federbelastete Element, an welchem die Rückhaltenase 191 angebracht ist, entgegen der Federkraft nach unten gedrückt werden.

Die vorstehenden Wandkanten der Vorderwand und der Rückwand 11 sowie die Aufnahmehaken 190 und die Rückhaltenase 191 bilden gemeinsam eine Behälteraufnahme 19 des Fluidsammelbehälters 5.

Das Pumpaggregatgehäuse 1 weist einen gehäuseseitigen Vakuumanschluss 17 auf, welcher beim Anbringen des Fluidsammelbehälters 5 an das Pumpaggregatgehäuse 1 an einen entsprechend am Fluidsammelbehälter 5 vorgesehenen, aber in den Figuren nicht dargestellten behälterseitigen Vakuumanschluss gekoppelt wird, so dass via den gehäuseseitigen und den behälterseitigen Vakuumanschluss im Inneren des Fluidsammelbehälters 5 ein Vakuum erzeugbar ist, um via eine in den Figuren nicht gezeigte Sekretleitung Köperfluide anzusaugen und im Fluidsammelbehälter 5 zu sammeln. Die Sekretleitung verbindet den Fluidsammelbehälter 5 mit einer Kavität oder Wunde eines Patienten, aus welcher Körperfluide abgesaugt werden sollen.

Innerhalb der ersten Seitenwand 12 ist ausserdem eine Adapteraufnahme 18 vorgesehen, welche zur Aufnahme eines in den Figuren nicht gezeigten Schlauchadapters dient. Der Schlauchadapter verbindet die Sekretleitung via einen am Fluidsammelbehälter 5 vorgesehenen, aber in den Figuren ebenfalls nicht dargestellten behälterseitigen Sekretanschluss mit dem Inneren des Fluidsammelbehälters 5.

In einem Innenraum 16 des Pumpaggregatgehäuses 1 ist ein Antriebsstrang 2 mit einem Motor 20 und einer mit dem Motor 20 verbundenen Motorwelle 21 untergebracht. Das Pumpaggregatgehäuse 1 bildet zusammen mit dem Antriebsstrang 2 sowie mit allenfalls weiteren im Innenraum 16 angeordneten Elementen eine Antriebseinheit.

Die Motorwelle 21 treibt mit einem ersten Endbereich direkt eine in der Figur 1a aus darstellerischen Gründen nicht gezeigte Membranpumpe an. Mittels der Membranpumpe, welche im Innenraum 16 des Pumpaggregatgehäuses 1 angeordnet ist, kann zur Absaugung von Körperfluiden durch die Sekretleitung am gehäuseseitigen Vakuumanschluss 17 ein Vakuum erzeugt werden. Mit einem zweiten Endbereich, welcher in der Figur 1 nicht sichtbar ist, ist die Motorwelle 21 mit einem Freilauf und/oder Getriebe 22 verbunden, welches die Motorwelle 21 mit einer Antriebswelle 23 verbindet. Die Antriebswelle 23, welche sich entlang der Rotationsachse der Motorwelle 21 erstreckt, ragt durch die erste Seitenwand 12 hindurch.

Auf der Aussenseite des Pumpaggregatgehäuses 1 ist ein Kopplungselement 24 drehfest am Ende der Antriebswelle 23 angebracht. Das Kopplungselement 24 weist einen umlaufenden Zahnkranz 240 mit in regelmässigen Abständen angeordneten und sich radial nach aussen hin erstreckenden Zähnen auf. Der Zahnkranz 240 ist direkt an der Aussenseite der ersten Seitenwand 12 angeordnet und kann sogar an dieser anliegen. Direkt anschliessend an den Zahnkranz 240 weist das Kopplungselement 24 einen zylindrischen Abschnitt 241 auf, welcher an seinem äusseren Ende in einen konischen Abschnitt 242 übergeht. Der konische Abschnitt 242, welcher das äussere Ende des Kopplungselements 24 bildet, verjüngt sich konisch nach aussen und somit zum Fluidsammelbehälter 5 hin.

Via das Kopplungselement 24 kann ein Pumpenkopf 30 einer im Fluidsammelbehälter 5 integrierten Peristaltikpumpeneinheit 30 (Figur 1c) angetrieben werden, wenn der Fluidsammelbehälter 5 bestimmungsgemäss am Pumpaggregatgehäuse 1 angebracht ist. Das Kopplungselement 24 dient also dazu, die von Motor 20 erzeugte Bewegung der Antriebswelle 23 auf den Pumpenkopf 30 der Peristaltikpumpeneinheit 30 zu übertragen.

Der Motor 20 dient somit sowohl zum Antreiben der Membranpumpe als auch zum Antreiben der Peristaltikpumpeneinheit 3. In einer alternativen Ausführungsform können selbstverständlich auch zwei separate Motoren im Innenraum 16 des Pumpaggregatgehäuses vorgesehen sein, wobei einer für den Antrieb der Membranpumpe und der andere für den Antrieb der Peristaltikpumpeneinheit 3 dient.

Der Fluidsammelbehälter 5 weist, wie es insbesondere in den Figuren 1a und 1b gut erkennbar ist, eine Vorderwand 50, eine in den Figuren nicht sichtbare Rückwand, zwei Seitenwände 52 und 53 sowie eine obere Wand 54 und eine ebenfalls nicht sichtbare untere Wand auf. Diese Wände werden durch ein aus einem opaken Material hergestellten Basisteil 55 und einem transluszenten Teil 56 gebildet. An derjenigen Seitenwand 52, welche ausschliesslich durch das transluszente Teil 56 gebildet wird, ist eine Füllstandskala 560 vorgesehen.

Diejenige Seitenwand 53, welche ausschliesslich durch das Basisteil 55 gebildet wird, weist auf ihrer Aussenseite eine zentral angeordnete, kreisflächenförmige Vertiefung 555 auf. In radialer Richtung wird die Vertiefung 555 im Wesentlichen umlaufend von einer Begrenzungswandung 556 begrenzt. Im Basisteil 55 sind ausserdem ein Einlasskanal 557 sowie ein Auslasskanal 558 ausgebildet, welche sich jeweils geradlinig und parallel zueinander von der oberen Wand 54 bis in die Vertiefung 555 hinein erstrecken.

Der Einlasskanal 557, der Auslasskanal 558 sowie die Begrenzungswandung 556 bilden gemeinsam eine Schlauchführung für einen Instillationsschlauch 6. Dieser ist derart in die Schlauchführung eingelegt, dass er sich durch den Einlasskanal 557 in die Vertiefung 555 hinein und dort entlang der Begrenzungswandung 556 zu einem überwiegenden Teil um den Pumpenkopf 30 herum erstreckt. Via den Auslasskanal 558 führt der Instillationsschlauch 6 wieder aus dem Fluidsammelbehälter 5 heraus. Um ein Verrutschen des Instillationsschlauchs 6 insbesondere entlang seiner Längsrichtung zu verhindern, ist an diesem in den Bereichen, wo der Einlasskanal 557 und der Auslasskanal 558 in der oberen Wand 54 nach aussen münden, jeweils ein Halteelement 559 fixiert. Die Halteelemente 559 sind in entsprechend dafür im Einlasskanal 557 und im Auslasskanal 558 vorgesehenen Ausnehmungen eingesetzt, so dass eine Längsverschiebung und eine Verdrehung des Instillationsschlauches 6 verunmöglicht wird.

An den Enden des Instillationsschlauches 6 sind jeweils Anschlusselemente 60 vorgesehen, welche zum Anschliessen von weiteren Schläuchen dienen. Beispielsweise kann eines der Anschlusselemente 60 via einen ersten weiteren Schlauch mit einem Behälter verbunden sein, in welchem die einem Patienten zuzuführende Substanz gelagert ist. Das andere Anschlusselement 60 kann dann zum Beispiel via einen zweiten weiteren Schlauch mit einem Wundbereich verbunden sein, so dass die Substanz mittels der Peristaltikpumpe 3 aus dem Behälter durch den Instillationsschlauch 6 hindurch zur Wunde befördert wird.

Innerhalb der Vertiefung 555 ist der Pumpenkopf 30 einer Peristaltikpumpeneinheit 3 angeordnet. Der Pumpenkopf 30 wird im vorliegenden Ausführungsbeispiel durch drei jeweils identisch ausgestaltete Rollenelemente 300 gebildet. Die Rollenelemente 300 werden jeweils durch Hohlräder gebildet, die flach in der Vertiefung 555 liegen und dort frei bewegbar sind.

Wie es insbesondere aus der Figur 1d gut ersichtlich ist, weisen die Rollenelemente 300 jeweils einen äusseren Zahnkranz 301 sowie eine radial nach aussen hin gerichtete zylindrische Fläche 302 auf. Die Rollenelemente 300, welche bevorzugt jeweils einstückig aus einem Kunststoff hergestellt sind, bilden die Satellitenräder der Peristaltikpumpe 3.

In der in der Figur 1d gezeigten Situation befindet sich der durch die Rollenelemente 300 gebildete Pumpenkopf in einem entkoppelten Zustand, in welchem das Kopplungselement 24 beabstandet zum Pumpenkopf 30 angeordnet ist. Mit anderen Worten ist der Fluidsammelbehälter 5 nicht am Pumpaggregatsgehäuse 1 angeschlossen. In diesem entkoppelten Zustand sind die Rollenelemente 300 innerhalb des vom Instillationsschlauch 6 seitlich begrenzten Raum der Vertiefung 555 frei beweglich. Insbesondere ist zwischen den einzelnen Rollenräder 300 keine Verbindung vorhanden, das heisst sie können beliebig und unabhängig voneinander gedreht und verschoben werden.

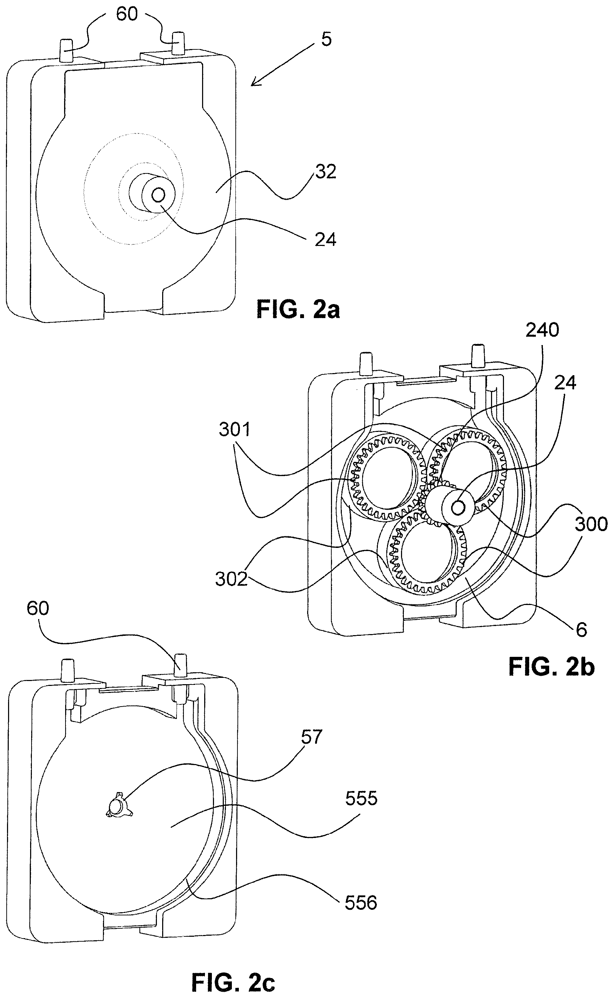

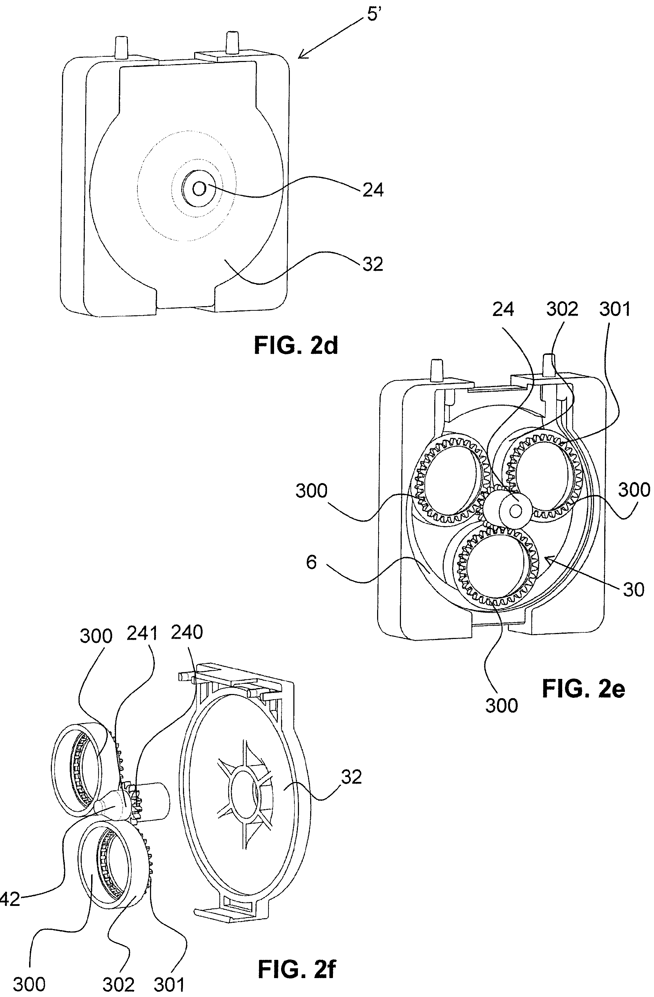

Die Rollenräder 300 sind in einem Dreieck angeordnet und liegen mit ihren zylindrischen Flächen 302 einerseits aneinander und andererseits am Instillationsschlauch 6 an. Dabei üben die Rollenräder 300 in dem in der Figur 1d gezeigten entkoppelten Zustand nur einen geringen Druck auf den Instillationsschlauch 6 aus. Es wäre auch denkbar, dass sie gar keinen Druck auf den Instillationsschlauch 6 ausüben und diesen im entkoppelten Zustand gar nicht berühren. Das Anliegen der Rollenräder 300 aneinander sowie am Instillationsschlauch 6 entsprechend der Figur 1d hat aber den Vorteil, dass dadurch ein Klappern bzw. gegenseitiges Aneinanderschlagen der Rollenräder 300 z.B. beim Transport des Fluidsammelbehälters verhindert wird. Alternativ oder zusätzlich können innerhalb der Vertiefung 555 auch Positionierelemente 57 vorgesehen sein, um die Rollenposition währen der Lagerung zu definieren (siehe Figur 2c).

Indem die Rollenräder im entkoppelten Zustand gar keinen oder nur einen geringen Druck auf den Instillationsschlauch 6 ausüben, werden Beschädigungen des Instillationsschlauchs 6 beim Transport oder bei der Lagerung vermieden. Weiters kann der Instillationsschlauch 6 in seinem Inneren komplett sterilisiert werden. Das Material des Instillationsschlauchs 6 ist unbelastet und weist dadurch eine grössere Lebensdauer auf. Die Anforderungen an den Instillationsschlauch 6 sind somit verringert, und er kann insbesondere dünnwandiger hergestellt werden, wodurch sich auch die Herstellungskosten verringern. Dasselbe gilt für die Rollenräder 300, an welche aufgrund des unbelasteten Zustandes ebenfalls geringere Anforderungen gelten, und die deshalb dünnwandiger und aus einem verhältnismässig günstigen und für den Spritzguss gut geeigneten Material, wie zum Beispiel Polypropylen (PP), hergestellt werden können. Eine dünnwandigere Ausbildung des Instillationsschlauches 6 führt zudem dazu, dass die vom Motor 20 via den Antriebsstrang 2 zu verrichtende Walk- bzw. Verformarbeit reduziert wird. Der Motor 20 kann somit mit einer geringeren Motorenleistung betrieben werden bzw. ein bzgl. seiner Leistung kleiner dimensionierter Motor 20 kann eingesetzt werden.

Beim Anschliessen des Fluidsammelbehälters 5 am Pumpaggregatgehäuse 1 wird das Kopplungselement 24, wie es in der Figur 1f gezeigt ist, zwischen die Rollenräder 300 geschoben. Der konische Abschnitt 242 drückt dabei die Rollenräder 300 jeweils in radialer Richtung nach aussen und gegen den Instillationsschlauch 6. Der Instillationsschlauch 6 wird dabei von den Rollenrädern 300 gegen die Begrenzungswandung 556 gepresst und verformt sich dadurch. Wenn das Kopplungselement 24 vollständig zwischen die Rollenräder 300 vorgeschoben ist, liegt der konische Abschnitt 242 des Kopplungselements 24 an den zylindrischen Flächen 302 der Rollenräder 300 an und hält die Rollenräder 300 dadurch in ihrer gegen den Instillationsschlauch 6 gepressten Position. Der Zahnkranz 240 des Kopplungselements 24 steht dabei in kämmendem Eingriff mit den äusseren Zahnkränzen 301 der Rollenräder 300. Dieser Zustand des Pumpenkopfes 30, wenn das Kopplungselement 24 vollständig zwischen die Rollenräder 300 vorgeschoben ist, wird als gekoppelter Zustand bezeichnet.

Aufgrund des Eingriffs des Zahnkranzes 240 mit den äusseren Zahnkränzen 301 wird eine vom Motor 20 verursachte Drehbewegung des Kopplungselements 24 direkt auf die Rollenräder 300 übertragen, welche dadurch mit ihren zylindrischen Flächen 302 auf dem Instillationsschlauch 6 abrollen und so die gewünschte Pumpwirkung der Peristaltikpumpe 3 bewirken. Aufgrund der gegenseitigen Verzahnung wird eine klar definierte Untersetzung der Drehbewegung des Kopplungselements 24 auf diejenige des Pumpenkopfes 30 bzw. der Rollenräder 300 erzielt.

Die Rollenräder 300 können im gekoppelten Zustand, wie es insbesondere in der Figur 1h erkennbar ist, aufgrund des Druckes zwischen dem Kopplungselement 24 und dem Instillationsschlauch 6 gegenüber dem entkoppelten Zustand elastisch verformt sein. Dies stellt keinen Nachteil dar, sondern kann sogar erwünscht sein. Aufgrund der leichten Verformung der Rollenräder 300, im vorliegenden Fall von einer kreisrunden zu einer leicht ovalen Form, entsteht eine federnde Wirkung. Es kann dadurch eine gleichmässige Anpresskraft auf den Instillationsschlauch 6 durch die verschiedenen Rollenräder 300 auch bei verhältnismässig grossen Produktionstoleranzen erzielt werden.

Wie es in den Figuren 1b, 1c und 1g erkennbar ist, weist der Fluidsammelbehälter 5 eine Abdeckung 32 auf, welche die Vertiefung 555 sowie die den Einlasskanal 557 und den Auslasskanal 558 abdeckt. In einem zentralen Bereich der Vertiefung 555 weist die Abdeckung 32 eine kreisrunde Aussparung auf, durch welche hindurch das Kopplungselement 24 hindurchragt, wenn der Fluidsammelbehälter 5 am Pumpaggregatgehäuse 1 angeschlossen ist.

In den Figuren 2a bis 2f ist eine weitere Ausführungsform eines Anschlussteils für eine erfindungsgemässe Vorrichtung gezeigt. Beim Anschlussteil handelt es sich hier um eine Peristaltikkassette 5', welche zum Beispiel als Zwischenteil zwischen einem Fluidsammelbehälter und dem Pumpaggregatgehäuse 1 angeordnet werden kann. Die Peristaltikkassette 5' weist ein Gehäuse mit einer Schlauchführung für die Instillationsleitung 6 auf, welche durch das Gehäuse hindurchgeführt ist. Vorteilhaft werden im Wesentlichen sämtliche Teile der Peristaltikkassette 5' im Spritzgussverfahren aus einem Kunststoff hergestellt.

Die Peristaltikkassette 5' hat insgesamt eine im Wesentlichen quaderförmige, dünne Aussenform. Das Gehäuse der Peristaltikkassette 5' weist, wie es insbesondere in der Figur 2c erkennbar ist, eine im Wesentlichen kreisförmige Vertiefung 555 auf, innerhalb welcher ein Pumpenkopf 30 einer Peristaltikpumpe 3 frei drehbar angeordnet ist (Figur 2b). Der Pumpenkopf 30 ist identisch ausgebildet wie derjenige der in den Figuren 1a bis 1h gezeigten Ausführungsform. Er weist ebenfalls mehrere Rollenräder 300 auf, welche dazu dienen, auf dem in der Vertiefung 555 um den Pumpenkopf 30 herumgelegten Instillationsschlauch 6 abzurollen und mittels mechanischer Verformung des Schlauches dabei eine Substanz durch den Instillationsschlauch 6 hindurch dem Wundbereich zuzuführen.

Im Gegensatz zu der in den Figuren 1 a bis 1h gezeigten Ausführungsform bildet das Kopplungselement 24 bei der Ausführungsform der Figuren 2a bis 2f einen Teil des Anschlussteils, hier also der Peristaltikkassette 5'. Die Abdeckung 32 weist im Bereich um die zentrale Aussparung eine konische Wölbung auf, welche auf der zur Vertiefung 555 hin gewandten Seite mittels Rippen verstärkt ist (siehe Figur 2f). Durch den Anschlag des Zahnkranzes 240 an diesen Rippen wird ein Herausfallen des Kopplungselements 24 aus der Peristaltikkassette 5' verunmöglicht. Um das Kopplungselement 24 in den mit dem Pumpenkopf 30 gekoppelten Zustand zu bringen, kann dieses einfach von aussen her durch die in der Abdeckung 32 vorgesehene Aussparung hindurch in die Peristaltikkassette 5' hineingedrückt werden. Auf seiner nach aussen hin gewandten Seite weist das Kopplungselement eine Eingriffsstruktur auf, welche mit einer Antriebseinheit in Eingriff bringbar ist, so dass der Pumpenkopf 30 via das Kopplungselement 24 von der Antriebseinheit in eine Drehbewegung versetzbar ist.

Die in den Figuren 1a bis 2f gezeigten Ausführungsformen können grundsätzlich eine beliebige Anzahl von Rollenrädern 300 aufweisen, also insbesondere auch nur ein einziges, zwei oder mehr als drei. Der zylindrische Abschnitt 241 des Kopplungselements 24 und die zylindrische Fläche 302 der Rollenelemente 300 bringen den Vorteil mit sich, dass die Anpresskraft der Rollenelemente 300 auf den Instillationsschlauch 6 nicht via die Zahnkränze 240 und 301 übertragen werden müssen. Prinzipiell können der zylindrische Abschnitt und die zylindrische Fläche 302 aber auch entfallen. Die in den Ausführungsformen der Figuren 1a bis 2f gezeigten Peristaltikpumpen 3 können in alternativen Ausführungsformen auch zum Absaugen von Fluiden aus einem menschlichen oder tierischen Körper dienen. Bei einer rein reibschlüssigen Momentübertragung können die Zahnkränze 240 und 301 entfallen.

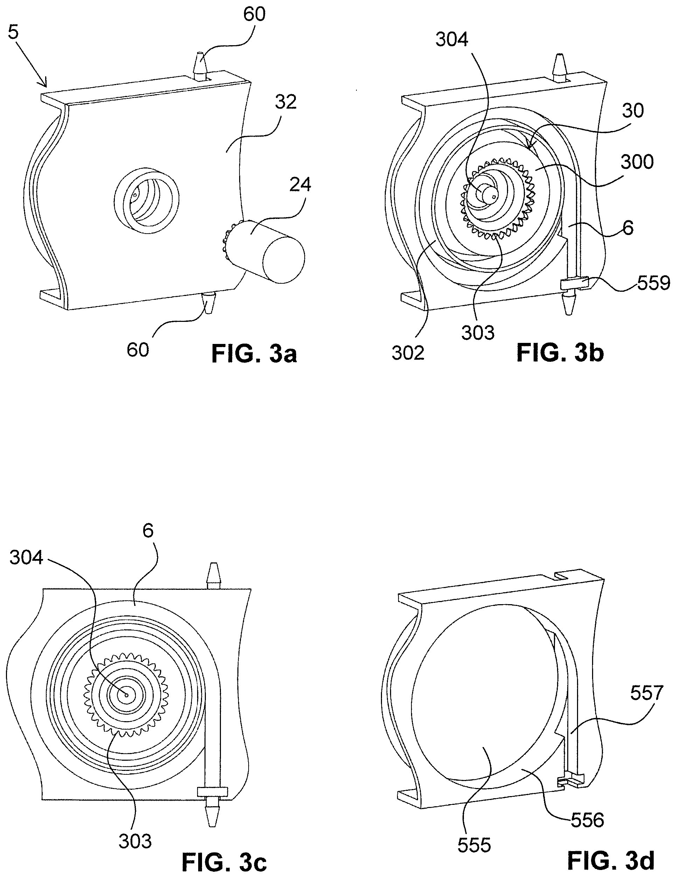

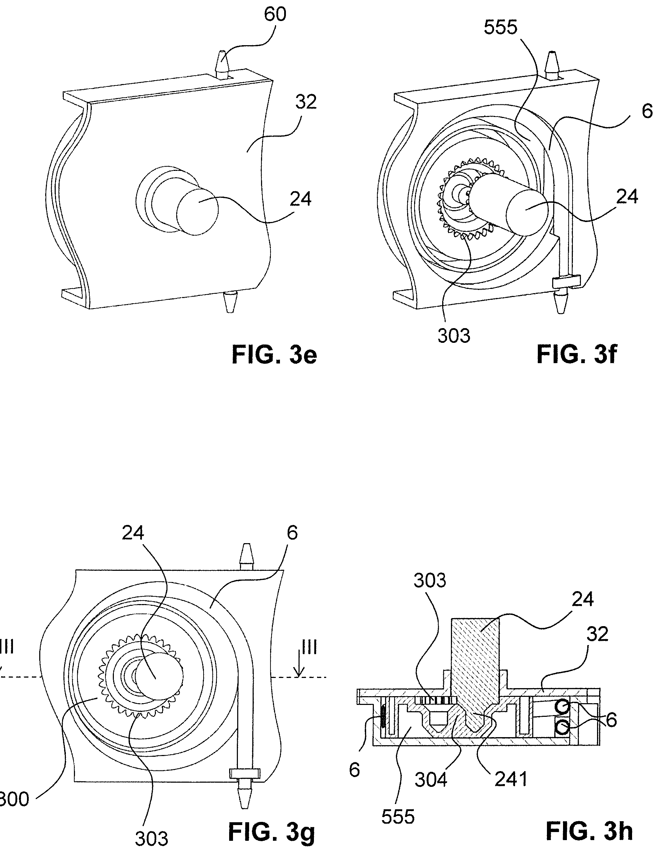

Ein Anschlussteil einer weiteren erfindungsgemässen Ausführungsform ist in den Figuren 3a bis 3h gezeigt. Beim Anschlussteil kann es sich beispielsweise um einen Fluidbehälter oder eine Peristaltikkassette handeln. Wenn es sich um einen Fluidbehälter handelt, kann dieser zum Sammeln von abgesaugten Fluiden oder zur Bereitstellung einer zuzuführenden Substanz dienen. Ebenso wie die in den Figuren 1a bis 2f gezeigten Ausführungsformen ist auch hier der Pumpenkopf 30 innerhalb einer kreisrunden Vertiefung 555 eines Gehäuses angeordnet. In die Vertiefung 555 eingelegt ist zudem auch hier ein Instillationsschlauch 6, welcher sich entlang der Begrenzungswandung 556 um den Pumpenkopf 30 herum erstreckt. Im Gegensatz zu den in den Figuren 1 a bis 3h gezeigten Ausführungsformen kreuzt sich der Instillationsschlauch 6 hier aber im Inneren des Anschlussteils und wird auf gegenüberliegenden Seiten aus diesem herausgeführt.

Die Fliessrichtung des Fluids durch den Einlasskanal 557 und den in den Figuren 3a-3h nicht sichtbaren Auslasskanal erstreckt sich entlang der Gravitationskraft von oben nach unten. Dadurch wird ein Zurückfliessen des Fluids verhindert. Durch die Mündung des Einlasskanales 557 und des Auslasskanales auf gegenüberliegenden Gehäuseseiten nach aussen hin bzw. aufgrund der Anordnung der Anschlusselemente 60 auf gegenüberliegenden Gehäuseseiten kann ein Abknicken des Schlauches bei der Schlauchverlegung durch das medizinische Fachpersonal eher vermieden werden.

Der Pumpenkopf 30 der Ausführungsform der Figuren 3a bis 3h weist nur ein einziges Rollenelement 300 auf. Wie es in der Figur 3c erkennbar ist, füllt dieses im entkoppelten Zustand den vom Instillationsschlauch 6 seitlich begrenzten Raum im Wesentlichen vollständig aus, ohne dabei einen wesentlichen Druck auf den Instillationsschlauch 6 auszuüben. Das Rollenelement 300 weist eine topfartige Ausgestaltung mit einer zentral angeordneten, kreisrunden Vertiefung auf. Die Vertiefung wird in ihrem Öffnungsbereich von einem inneren Zahnkranz 303 umrandet, welcher eine Vielzahl von in regelmässigen Abständen angeordneten und sich radial nach innen erstreckenden Zähnen aufweist. Das Rollenrad 300 kann somit auch hier als ein Hohlrad bezeichnet werden.

Zentral innerhalb der vom inneren Zahnkranz 303 umrandeten Vertiefung weist das Rollenrad 300 einen Umlaufnocken 304 auf. Der Umlaufnocken 304 hat eine zylindrische Aussenfläche, an welche ein sich konisch verjüngender Endabschnitt anschliesst.

Das Rollenrad 300 weist eine zylindrische Aussenfläche 302 auf, die durch einen umlaufenden Randbereich des Rollenrades 30 gebildet wird (siehe Figur 3b). Zwischen dem Randbereich und einem inneren Bereich, an dessen Innenseite der innere Zahnkranz 303 ausgebildet ist, weist das Rollenrad 300 eine umlaufende Vertiefung auf. Aufgrund dieser umlaufenden Vertiefung ist die zylindrische Aussenfläche 302 zu einem gewissen Grad elastisch verformbar.

Beim Ankoppeln des Kopplungselements 24, welches Teil des Anschlussteils sein kann aber nicht muss, wird dieses durch eine in der Abdeckung 24 vorgesehenen Aussparung hindurch und in die zentrale kreisrunde Vertiefung des Rollenelements 300 hinein vorgeschoben. Das Kopplungselement 24 gleitet dabei mit seinem konischen Abschnitt 242 am konischen Endabschnitt des Umlaufnockens 304 entlang und drückt das Rollenelement 300 dadurch in radialer Richtung zum Instillationsschlauch 6 hin. Im vollständig gekoppelten Zustand liegt das Kopplungselement 24, wie es in der Figur 3h erkennbar ist, mit seinem zylindrischen Abschnitt 241 seitlich an der zylindrischen Aussenfläche des Umlaufnockens 304 an, wodurch das Rollenrad 300 in seiner Position gehalten und der Instillationsschlauch 6 zusammengequetscht wird. Das Rollenrad 300 kann sich dabei im Bereich seiner zylindrischen Aussenfläche 302 aufgrund der umlaufenden Vertiefung leicht verformen, wie dies in den Figuren 3g und insbesondere 3h erkennbar ist.

Im gekoppelten Zustand steht ausserdem der äussere Zahnkranz 240 des Kopplungselements 24 in kämmendem Eingriff mit dem inneren Zahnkranz 303 des Rollenelements 300. Dadurch wird eine Drehbewegung des Kopplungselements 24 direkt auf das Rollenelement 300 übertragen, so dass im gekoppelten Zustand mittels eines externen Antriebs der Pumpenkopf 30 via das Kopplungselement 24 in eine Drehbewegung versetzbar ist. Der durch das Rollenelement 300 gebildete Pumpenkopf 30 rollt dabei auf dem Instillationsschlauch 6 ab und erzeugt dadurch die erwünschte Pumpwirkung.

Die in den Figuren 3a bis 3h gezeigte Ausführungsform hat den besonderen Vorteil, dass die Übertragung der Anpresskraft vom Kopplungselement 25 auf den Pumpenkopf 30 beabstandet zu derjenigen Stelle bewirkt wird, wo der Zahnkranz 240 des Kopplungselements 24 mit dem inneren Zahnkranz 303 des Rollenelements 300 kämmt. Die Übertragung der radialen Anpresskraft einerseits und die Übertragung des Drehmoments andererseits sind also örtlich voneinander getrennt. Zudem kann hier der Pumpenkopf 30 aus einem einzigen, vorteilhaft einstückig ausgebildeten Rollenelement 300 ausgebildet sein, dessen radiale Ausdehnung innerhalb der Vertiefung 555 ausserdem maximiert werden kann.

### BEZUGSZEICHENLISTE

| | | | |
|---|---|---|---|
| 1 | Pumpaggregatgehäuse | 300 | Rollenelement |
| 11 | Rückwand | 301 | Äusserer Zahnkranz |
| 12 | Seitenwand | 302 | Zylindrische Fläche |
| 13 | Seitenwand | 303 | Innerer Zahnkranz |
| 14 | Obere Wand | 304 | Umlaufnocken |
| 15 | Untere Wand | 32 | Abdeckung |
| 16 | Innenraum | | |
| 17 | Gehäuseseitiger | 5 | Fluidsammelbehälter |
| | Vakuumanschluss | 5' | Peristaltikkassette |
| 18 | Adapteraufnahme | 50 | Vorderwand |
| 19 | Behälteraufnahme | 52 | Seitenwand |
| 190 | Aufnahmehaken | 53 | Seitenwand |
| 191 | Rückhaltenase | 54 55 | Obere Wand Basisteil |
| 2 | Antriebsstrang | 554 | Zapfen |
| 20 | Motor | 555 | Vertiefung |
| 21 | Motorwelle | 556 | Begrenzungswandung |
| 22 | Freilauf/Getriebe | 557 | Einlasskanal |
| 23 | Antriebswelle | 558 | Auslasskanal |
| 24 | Kopplungselement | 559 | Halteelement |
| 240 | Zahnkranz | 56 | Transluszentes Teil |
| 241 | Zylindrischer Abschnitt | 560 | Füllstandskala |
| 242 | Konischer Abschnitt | 57 | Positionierelement |
| 3 | Peristaltikpumpeneinheit | 6 | Instillationsschlauch |
| 30 | Pumpenkopf | 60 | Anschlusselemente |

## Patentansprüche

1. Vorrichtung zum Zuführen und/oder Absaugen einer fluiden Substanz zu oder von einem menschlichen oder tierischen Körper, aufweisend
eine Antriebseinheit (1, 2) mit einem Antrieb (20);
ein an die Antriebseinheit (1, 2) anschliessbares Anschlussteil (5; 5'), welches eine Peristaltikpumpeneinheit (3) mit einem Pumpenkopf (30), einer Schlauchführung (556, 557, 558) sowie einem in der Schlauchführung (556, 557, 558) eingelegten Schlauch (6) aufweist, wobei der Pumpenkopf (30) eines oder mehrere Rollenelemente (300) aufweist, welche zum Abrollen auf dem Schlauch (6) dienen, um dadurch die fluide Substanz durch den Schlauch (6) hindurch zu befördern; sowie
ein mit dem Pumpenkopf (30) koppelbares Kopplungselement (24), welches bei bestimmungsgemässem Anschluss des Anschlussteils (5; 5') an die Antriebseinheit (1, 2) zur Übertragung einer Bewegung vom Antrieb (20) auf den Pumpenkopf (30) dient,
wobei der Pumpenkopf (30) einen vom Kopplungselement (24) entkoppelten Zustand aufweist, in welchem das oder die Rollenelemente (300) keinen oder einen vergleichsweise geringen mechanischen Druck auf den Schlauch (6) ausüben, und zudem einen mit dem Kopplungselement (24) gekoppelten Zustand aufweist, in welchem das oder die Rollenelemente (300) vom Kopplungselement (24) mit einem vergleichsweise grossen mechanischen Druck gegen den Schlauch (6) gepresst werden,
und wobei das oder die Rollenelemente (300) im entkoppelten Zustand innerhalb eines Bereiches (555) frei und unabhängig voneinander bewegbar sind, der von dem in die Schlauchführung (556, 557, 558) eingelegten Schlauch (6) seitlich begrenzt ist,
**dadurch gekennzeichnet, dass**
das Kopplungselement (24) einen ersten Verzahnungsbereich (240) aufweist, und dass das oder die Rollenelemente (300) jeweils einen zweiten Verzahnungsbereich (301; 303) aufweisen, welche im gekoppelten Zustand derart in den ersten Verzahnungsbereich (240) eingreifen, dass eine Bewegung des Kopplungselements (24) direkt auf das oder die Rollenelemente (300) übertragen wird.

2. Vorrichtung nach Anspruch 1, wobei der Pumpenkopf (30) mehrere Rollenelemente (300) aufweist, welche sich im entkoppelten Zustand gegenseitig berühren können.

3. Vorrichtung nach einem der vorhergehenden Ansprüche, wobei das oder die Rollenelemente (300) im entkoppelten Zustand jeweils ein gewisses seitliches Spiel haben.

4. Vorrichtung nach einem der vorhergehenden Ansprüche, wobei das oder die Rollenelemente (300) im gekoppelten Zustand jeweils derart vom Kopplungselement (24) gegen den Schlauch (6) gepresst werden, dass sie sich im Vergleich zum entkoppelten Zustand für das menschliche Auge gut sichtbar verformen.

5. Vorrichtung nach einem der vorhergehenden Ansprüche, wobei das Kopplungselement (24) einen konisch ausgebildeten Abschnitt (242) aufweist.

6. Vorrichtung nach einem der vorhergehenden Ansprüche, wobei das Kopplungselement (24) einen zylindrischen Abschnitt (241) aufweist, welcher im gekoppelten Zustand zum Pressen des oder der Rollenelemente (300) gegen den Schlauch (6) dient.

7. Vorrichtung nach einem der vorhergehenden Ansprüche, wobei das Kopplungselement (24) ein Teil der Antriebseinheit (1, 2) ist.

8. Vorrichtung nach einem der Ansprüche 1 bis 6, wobei das Kopplungselement (24) ein Teil des Anschlussteils (5') ist.

9. Vorrichtung nach Anspruch 8, wobei es sich beim Anschlussteil (5) um einen Fluidbehälter zum Sammeln oder Bereitstellen eines Fluids handelt.

10. Vorrichtung nach einem der vorhergehenden Ansprüche, wobei das oder die Rollenelemente (300) jeweils durch ein Hohlrad gebildet werden.

11. Vorrichtung nach Anspruch 10, wobei der zweite Verzahnungsbereich durch jeweils einen am Hohlrad ausgebildeten äusseren Zahnkranz (301) gebildet wird.

12. Vorrichtung nach Anspruch 10, wobei der Pumpenkopf (30) ein einziges Rollenelement (300) aufweist, das durch ein Hohlrad gebildet wird, und wobei es sich beim zweiten Verzahnungsbereich um einen am Hohlrad ausgebildeten inneren Zahnkranz (303) handelt.

13. Vorrichtung nach einem der vorhergehenden Ansprüche, wobei der Pumpenkopf (30) als Ganzes im Spritzgussverfahren aus einem Kunststoff hergestellt ist.

14. Anschlussteil (5; 5') einer Vorrichtung zum Zuführen und/oder Absaugen einer fluiden Substanz zu oder von einem menschlichen oder tierischen Körper, insbesondere einer Vorrichtung nach einem der vorhergehenden Ansprüche, aufweisend eine Peristaltikpumpeneinheit (3) mit einem Pumpenkopf (30), einer Schlauchführung (556, 557, 558) sowie einem in der Schlauchführung (556, 557, 558) eingelegten Schlauch (6),
wobei der Pumpenkopf (30) eines oder mehrere Rollenelemente (300) aufweist, welche zum Abrollen auf dem Schlauch (6) dienen, um dadurch die fluide Substanz durch den Schlauch (6) hindurch zu befördern,
wobei der Pumpenkopf (30) mit einem Kopplungselement (24) koppelbar ist, welches zur Übertragung einer Bewegung von einem Antrieb (20) auf den Pumpenkopf (30) dient,
wobei das oder die Rollenelemente (300) in einem vom Kopplungselement (24) entkoppelten Zustand keinen oder einen vergleichsweise geringen mechanischen Druck auf den Schlauch ausüben, und aber mit dem Kopplungselement (24) derart koppelbar sind, dass sie in einem gekoppelten Zustand vom Kopplungselement (24) mit einem vergleichsweise grossen mechanischen Druck gegen den Schlauch (6) gepresst werden,
und wobei das oder die Rollenelemente (300) im entkoppelten Zustand innerhalb eines Bereiches (555) frei und unabhängig voneinander bewegbar sind, der von dem in die Schlauchführung (556, 557, 558) eingelegten Schlauch (6) seitlich begrenzt ist
**dadurch gekennzeichnet, dass**
das oder die Rollenelemente (300) jeweils einen zweiten Verzahnungsbereich (301; 303) aufweisen, welcher im gekoppelten Zustand zum Eingreifen in einen ersten Verzahnungsbereich (240) des Kopplungselements (24) ausgebildet ist.

## Claims

1. Device for supplying and/or aspirating a fluid substance to or from a human or animal body, which device has
a drive unit (1, 2) with a drive (20);
an attachment part (5; 5') which is attachable to the drive unit (1, 2) and which has a peristaltic pump unit (3) with a pump head (30), a hose guide (556, 557, 558), and a hose (6) placed in the hose guide (556, 557, 558), wherein the pump head (30) has one or more roller elements (300) which serve for rolling on the hose (6) in order thereby to convey the fluid substance through the hose (6); and
a coupling element (24) which can be coupled to the pump head (30) and which serves to transmit a movement from the drive (20) to the pump head (30) when the attachment part (5; 5') is attached as intended to the drive unit (1, 2), wherein the pump head (30) has a state in which it is decoupled from the coupling element (24), and in which the one or more roller elements (300) apply no mechanical pressure or a comparatively low mechanical pressure to the hose (6), and in addition a state in which it is coupled to the coupling element (24), and in which the one or more roller elements (300) are pressed by the coupling element (24) against the hose (6) with a comparatively high mechanical pressure,
wherein the one or more roller elements (300), in the decoupled state, are movable freely and independently of each other within a range (555) which is laterally limited by the hose (6) placed in the hose guide (556, 557, 558)
**characterized in that**
the coupling element (24) has a first toothing area (240), and the one or more roller elements (300) each have a second toothing area (301; 303) engaging, in the coupled state, in the first toothing area (240) in such a way that a movement of the coupling element (24) is transmitted directly to the one or more roller elements (300).

2. Device according to Claim 1, wherein the pump head (30) has several roller elements (300) which are able to touch each other in the decoupled state.

3. Device according to any one of the preceding Claims, wherein the one or more roller elernents (300) each have a lateral play in the decoupled state.

4. Device according to any one of the preceding Claims, wherein the one or more roller elements (300) in the coupled state are each pressed against the hose (6) by the coupling element (24) in such a way that, by comparison with the decoupled state, they deform in a way that is clearly visible to the human eye.

5. Device according to any one of the preceding Claims, wherein the coupling element (24) has a conically shaped portion (242).

6. Device according to any one of the preceding Claims, wherein the coupling element (24) has a cylindrical portion (241) which, in the coupled state, serves to press the one or more roller elements (300) against the hose (6).

7. Device according to any one of the preceding Claims, wherein the coupling element (24) is a part of the drive unit (1, 2).

8. Device according to one of Claims 1 to 6, wherein the coupling element (24) is a part of the attachment part (5').

9. Device according to Claim 8, wherein the attachment part (5) is a fluid container for collecting or making available a fluid.

10. Device according to any one of the preceding Claims, wherein the one or more roller elements (300) are each formed by a hollow wheel.

11. Device according to Claim 10, wherein the second toothing area is formed by an outer toothed ring (301) provided in each case on the hollow wheel.

12. Device according to Claim 10, wherein the pump head (30) has a single roller element (300), which is formed by hollow wheel, and wherein the second toothing area is an inner toothed ring (303) provided on the hollow wheel.

13. Device according to any one of the preceding Claims, wherein the pump head (30) is produced as a whole from plastic in an injection moulding method.

14. Attachment part (5; 5') of a device for supplying and/or aspirating a fluid substance to or from a human or animal body, in particular of a device according to one of the preceding Claims, having a peristaltic pump unit (3) with a pump head (30), a hose guide (556, 557, 558), and a hose (6) placed in the hose guide (556, 557, 558),
wherein the pump head (30) has one or more roller elements (300) which serve for rolling on the hose (6) in order thereby to convey the fluid substance through the hose (6),
wherein the pump head (30) can be coupled to a coupling element (24) which serves to transmit a movement from a drive (20) to the pump head (30), wherein, in a state decoupled from the coupling element (24), the one or more roller elements (300) apply no mechanical pressure or a comparatively low mechanical pressure to the hose (6), but can be coupled to the coupling element (24) in such a way that, in a coupled state, the one or more roller elements (300) are pressed by the coupling element (24) against the hose (6) with a comparatively high mechanical pressure, and
wherein the one or more roller elements (300), in the decoupled state, are movable freely and independently of each other within a range (555) which is laterally limited by the hose (6) placed in the hose guide (556, 557, 558)
**characterized in that**
the one or more roller elements (300) each have a second toothing area (301; 303) configured for engaging, in the coupled state, in a first toothing area (240) of the coupling element (24).

## Revendications

1. Dispositif pour l'alimentation et/ou l'aspiration d'une substance fluide vers ou depuis un corps humain ou animal, comprenant
une unité d'entraînement (1, 2) avec un entraînement (20) ;
une pièce de raccordement (5 ; 5') pouvant être raccordée à l'unité d'entraînement (1, 2) et présentant une unité de pompe péristaltique (3) avec une tête de pompe (30), un guide de tuyau (556, 557, 558) et un tuyau (6) inséré dans le guide de tuyau (556, 557, 558), dans lequel la tête de pompe (30) présente un ou plusieurs élément(s) formant rouleau (300) qui ser(ven)t à rouler sur le tuyau (6) afin de transporter la substance fluide à travers le tuyau (6) ; et
un élément de couplage (24) pouvant être couplé à la tête de pompe (30) et servant à transmettre un mouvement de l'entraînement (20) à la tête de pompe (30) lorsque la pièce de raccordement (5 ; 5') est raccordée de manière appropriée à l'unité d'entraînement (1, 2),
dans lequel la tête de pompe (30) présente un état découplé de l'élément de couplage (24), au sein duquel le ou les élément(s) formant rouleau (300) n'exerce(nt) aucune pression mécanique ou une pression mécanique relativement faible sur le tuyau (6), et présente également un état couplé à l'élément de couplage (24), au sein duquel le ou les élément(s) de roulage (300) est/sont pressé(s) contre le tuyau (6) par l'élément de couplage (24) avec une pression mécanique relativement élevée,
et dans lequel, à l'état découplé, le ou les élément(s) formant rouleau (300) peu(ven)t être déplacé(s) librement et indépendamment les uns des autres à l'intérieur d'une région (555) délimitée latéralement par le tuyau (6) inséré dans le guide de tuyau (556, 557, 558),
**caractérisé en ce que**
l'élément de couplage (24) présente une première région de denture (240), et **en ce que** le ou les élément(s) formant rouleau (300) présente(nt) respectivement une seconde région de denture (301 ; 303) qui, à l'état couplé, vient en prise dans la première région de denture (240) de telle manière qu'un mouvement de l'élément de couplage (24) est transmis directement à l'élément ou aux éléments formant rouleau (300).

2. Dispositif selon la revendication 1, dans lequel la tête de pompe (30) présente plusieurs éléments formant rouleau (300) qui peuvent se toucher à l'état découplé.

3. Dispositif selon l'une quelconque des revendications précédentes, dans lequel le ou les éléments formant rouleau (300) présente(nt) respectivement un certain jeu latéral à l'état découplé.

4. Dispositif selon l'une quelconque des revendications précédentes, dans lequel le ou les élément(s) formant rouleau (300) à l'état couplé est/sont pressé(s) contre le tuyau (6) par l'élément de couplage (24) de telle manière qu'il(s) se déforme(nt) de manière bien visible pour l'œil humain en comparaison avec l'état découplé.

5. Dispositif selon l'une quelconque des revendications précédentes, dans lequel l'élément de couplage (24) présente une partie conique (242).

6. Dispositif selon l'une quelconque des revendications précédentes, dans lequel l'élément de couplage (24) présente une partie cylindrique (241) qui, à l'état couplé, sert à presser le ou les élément(s) formant rouleau (300) contre le tuyau (6).

7. Dispositif selon l'une quelconque des revendications précédentes, dans lequel l'élément de couplage (24) fait partie de l'unité d'entraînement (1, 2).

8. Dispositif selon l'une quelconque des revendications 1 à 6, dans lequel l'élément de couplage (24) fait partie de la pièce de raccordement (5').

9. Dispositif selon la revendication 8, dans lequel la pièce de raccordement (5) est un réservoir de fluide permettant de collecter ou fournir un fluide.

10. Dispositif selon l'une quelconque des revendications précédentes, dans lequel le ou les élément(s) formant rouleau (300) est/sont formé(s) respectivement par une couronne dentée.

11. Dispositif selon la revendication 10, dans lequel la deuxième région de denture est formée par respectivement une couronne dentée extérieure (301) réalisée au niveau de la couronne dentée.

12. Dispositif selon la revendication 10, dans lequel la tête de pompe (30) présente un unique élément formant rouleau (300) formé par une couronne dentée, et dans lequel la deuxième région de denture est une couronne dentée intérieure (303) réalisée au niveau de la couronne dentée.

13. Dispositif selon l'une quelconque des revendications précédentes, dans lequel la tête de pompe (30) dans son ensemble est fabriquée à partir de plastique selon le procédé de moulage par injection.

14. Pièce de raccordement (5 ; 5') d'un dispositif d'alimentation et/ou d'aspiration d'une substance fluide vers ou depuis un corps humain ou animal, en particulier d'un dispositif selon l'une quelconque des revendications précédentes, présentant une unité de pompe péristaltique (3) avec une tête de pompe (30), un guide de tuyau (556, 557, 558) et un tuyau (6) inséré dans le guide de tuyau (556, 557, 558),
dans laquelle la tête de pompe (30) présente un ou plusieurs élément(s) formant rouleau (300) qui ser(ven)t à rouler sur le tuyau (6) afin de transporter la substance fluide à travers le tuyau (6),
dans laquelle la tête de pompe (30) peut être couplée à un élément de couplage (24) qui sert à transmettre un mouvement d'un entraînement (20) à la tête de pompe (30), dans laquelle le ou les élément(s) formant rouleau (300) n'exerce(nt) aucune pression mécanique ou une pression mécanique relativement faible sur le tuyau dans un état découplé de l'élément de couplage (24), mais peu(ven)t être couplé(s) à l'élément de couplage (24) de telle manière que, dans un état couplé, il(s) est/sont pressé(s) contre le tuyau (6) par l'élément de couplage (24) avec une pression mécanique relativement élevée,
et dans laquelle, à l'état découplé, le ou les élément(s) formant rouleau (300) peu(ven)t être déplacé(s) librement et indépendamment les uns des autres à l'intérieur d'une région (555) délimitée latéralement par le tuyau (6) inséré dans le guide de tuyau (556, 557, 558),
**caractérisée en ce que**
le ou les élément(s) formant rouleau (300) présente(nt) respectivement une deuxième région de denture (301 ; 303) qui, à l'état couplé, est conçue pour venir en prise dans une première région de denture (240) de l'élément de couplage (24).
